# EUROPEAN PATENT APPLICATION

(11) **EP 4 212 160 A1**
(43) Date of publication of application: **19.07.2023**
(21) Application number: 21865928.2
(22) Date of filing: 03.09.2021
(51) Int. Cl.: A61K 31/47, A61K 39/395, A61P 35/00

(54) **USE OF CHIAURANIB IN COMBINATION WITH IMMUNE CHECKPOINT INHIBITOR IN ANTITUMOR THERAPY**

(30) Priority: 09.09.2020 CN 202010939961
(71) Applicant: Shenzhen Chipscreen Biosciences Co., Ltd., Guangdong 518057 (CN); Chengdu Chipscreen Pharmaceutical Ltd., Chengdu, Sichuan 610000 (CN)
(72) Inventor: LU, Xianping, Shenzhen, Guangdong 518057 (CN); PAN, Desi, Shenzhen, Guangdong 518057 (CN); ZHOU, You, Shenzhen, Guangdong 518057 (CN)
(74) Representative: J A Kemp LLP
(86) International application number: PCT/CN2021/116498
(87) International publication number: WO 2022/052874

(57) **Abstract**

The present invention relates to the field of medicines, and in particular relates to use of chiauranib in combination with an immune checkpoint inhibitor in antitumor therapy. Use of a combination of chiauranib or a derivative thereof and the immune checkpoint inhibitor in the preparation of a medicine for treating a tumor, a pharmaceutical composition and a kit comprising the combination, and a method for treating a tumor by administering a therapeutically effective amount of the pharmaceutical composition or the kit to a tumor patient in need thereof. The combination has a synergistic effect and is suitable for treating a cancer.

## Description

### FIELD

The present invention relates to the field of medicines, and specifically, to the use of chiauranib in combination with an immune checkpoint inhibitor in antitumor therapy.

### BACKGROUND

Tumor is a major disease that threatens human health, and the research on tumor treatment has been widely concerned. Conventional tumor therapies include surgery, radiotherapy, chemotherapy, and the like. With the progress of medical science, novel therapies, such as targeted therapy and immunotherapy, are constantly emerging, providing new treatment options for cancer patients.

Targeted therapy is a treatment method using a corresponding targeted drug designed for an identified carcinogenic site at the cellular molecular level. After entering the human body, the targeted drug will specifically select the carcinogenic site to bind and take effect, causing specific death of tumor cells without affecting normal tissue cells around the tumor. Targeted therapy is currently the focus of the development of many anticancer drugs and is the cornerstone of precision medicine.

Chiauranib is a small molecule antitumor targeted drug targeting multiple protein kinases, and is an original compound that is independently developed by Shenzhen Chipscreen Biosciences Co., Ltd. with complete intellectual property rights. Due to the high selective inhibitory activity of chiauranib to VEGFR/PDGFR/c-Kit, Aurora B, and CSF-1R targets, chiauranib has the triple-pathway antitumor synergistic effect mechanism such as antitumor angiogenesis, inhibition of tumor cell mitosis, and regulation of tumor inflammatory microenvironment, and exerts a comprehensive antitumor effect.

CN200910223861.5 discloses a chiauranib compound, and specifically discloses a naphthamide derivative, and preparation method and use thereof. The compound has both the protein kinase inhibition activity and histone deacetylase inhibition activity, and may be used for treating diseases associated with abnormal protein kinase activity or abnormal histone deacetylase activity, including inflammation, autoimmune diseases, cancer, nervous system diseases and neurodegenerative diseases, cardiovascular diseases, metabolic diseases, allergies, asthma, and hormone-associated diseases.

CN201610856945.2 discloses non-solvated crystals A, B, and C of chiauranib and a preparation method thereof, and also relates to a pharmaceutical composition including the crystals, and the use of the crystals in the preparation of a drug for treating a disease associated with abnormal protein kinase activity or abnormal histone deacetylase activity.

CN201811550290.1 discloses new use of chiauranib in the preparation of a drug for treating acute myeloid leukemia, and the new use is use of chiauranib or a pharmaceutically acceptable salt, ester or solvate thereof in the preparation of a drug for preventing and/or treating acute myeloid leukemia. Chiauranib inhibits growth and induces apoptosis and colony formation of acute myeloid leukemia cells through the Src/Fyn/p38 and Erk/MEK signaling pathways.

Immunotherapy aims to activate the human immune system and relies the autoimmunity to kill cancer cells and tumor tissues. An immune checkpoint molecule, such as PD-1, is mainly expressed on the surface of an activated T lymphocyte. When its ligand PD-L1 or PD-L2 binds to PD-1, it conducts an immunosuppressive signal, so as to make the T lymphocyte in an immune tolerance or immunocompromised state. After the foregoing signaling pathway is blocked with an anti-PD-1 or PD-L1 antibody, the immunosuppressive signal of the T lymphocyte is released and further activated to exert antitumor activities such as killing and cytotoxicity. Therefore, immune checkpoint inhibitors, such as PD-1 inhibitors and PD-L1 inhibitors, have been approved for clinic treatment of a variety of tumors.

Both targeted therapy drugs and immune checkpoint inhibitors can show certain efficacy in clinical tumor treatment, and there are more and more studies on the combined use of the two.

WO2015088847A1 discloses use of a combination of Pazopanib and a PD-1 inhibitor. The animal experiment data and results of phase I/II clinical trials described in the description thereof show that the combination of the two has significant efficacy on tumors.

CN105960415A discloses use of a combination of axitinib and a PD-1 inhibitor. The clinical trial schemes described in the description thereof show that the combination of the two is expected to have significant efficacy on renal cell carcinoma.

WO2016141218A discloses use of a combination of lenvatinib and a PD-1 inhibitor. The animal experiment data described in the description thereof show that the combination of the two has significant efficacy on cancer.

CN108601831B discloses use of a combination of apatinib and a PD-1 inhibitor. The animal experiment data and results of phase I/II clinical trials described in the description thereof show that the combination of the two has significant efficacy on cancer.

CN106963948A discloses use of a combination of apatinib and a PD-1 inhibitor. The animal experiment data described in the description thereof show that the combination of the two has significant efficacy on colon cancer.

WO2019096194A1 discloses use of a combination of apatinib and a PD-1 inhibitor. The results of phase II clinical trials described in the description thereof show that the combination of the two has significant efficacy on small cell lung cancer.

CN109893654A discloses use of a combination of apatinib, a PD-1 inhibitor, and an IDO inhibitor. The animal experiment data described in the description thereof show that the combination of the three has significant efficacy on colon cancer.

Despite the impressive efficacy of the combinations of the targeted therapy drugs and the immune checkpoint inhibitors, the following two problems remain:
First, due to the complex mechanism and process of tumor targeted therapy and tumor immunotherapy, it is difficult to predict the efficacy of the combination of a targeted drug and an immune checkpoint inhibitor. Therefore, it is of great significance to extensively try combined therapy to further improve the applicability rate and benefit rate of a combination of drugs for cancer patients, reduce doses of single drugs, improve the incidence rate and/or severity of adverse events (AEs) during treatment, enhance the clinical control of tumor symptoms/progression, and prolong the degree and duration of the patient's response to the drugs, which is also a major challenge in the field of cancer treatment.
Second, use of a variety of combinations of VEGFR inhibitors and PD-1 inhibitors in tumor therapy has been disclosed in the prior art, but chiauranib is not a conventional VEGFR targeted inhibitor. Compared with conventional VEGFR targeted inhibitors, such as sunitinib and sorafenib, chiauranib has the unique inhibitory activity to Aurora B that is a key enzyme in mitosis, and has potential effects in reducing instability of tumor tissue genome and inhibiting tumor cell metastasis. Chiauranib exerts antitumor activity through three complementary mechanisms of action: inhibition of tumor angiogenesis, cell mitosis, and tumor inflammatory microenvironment, so it is worthy of making further study on whether a combination of chiauranib and an immune checkpoint inhibitor can produce a synergistic effect to improve the efficacy.

### SUMMARY

In order to solve the foregoing technical problems, the present invention proposes use of the combination of chiauranib or a derivative thereof and an immune checkpoint inhibitor in the preparation of a drug for treating a tumor.

The term *"treatment"* refers to any success or improvement in the progression, severity and/or duration of a disease, pathology or condition, including any objective or subjective parameters, such as reducing, remitting or alleviating symptoms or making an injury, pathology or condition more tolerable to a patient, slowing the rate of degeneration or decline to make a final point of degeneration less debilitating, and improving the physical and mental health of the patient.

The term *"inhibition"* refers to reduction of the activity, binding or expression of a polypeptide or relief and alleviation of a disease, disorder or condition or a symptom thereof. The inhibition described in the present invention may include partially or completely blocking stimulation, reducing, preventing or delaying activation or binding, or inactivating, desensitizing or downregulating protein or enzyme activity or binding.

Conventional effective amounts of the chiauranib or the derivative thereof and the immune checkpoint inhibitor described in the present invention are used.

The term *"effective amount"* refers to an amount of drug used in the course of treatment that is sufficient to achieve the therapeutic purpose or therapeutic effect. The effective amount may be sufficient to reduce and/or improve the progression, recurrence, severity and/or duration of a given disease, disorder or condition and/or an associated symptom thereof, and/or improve or enhance a prophylactic or therapeutic effect of another therapy. In the combination described in the present invention, an effective amount of therapeutic drug may enhance the efficacy of another therapeutic drug.

The chiauranib derivative includes a pharmaceutically acceptable salt thereof and non-solvated crystals A, B, and C thereof.

The non-solvated crystals A, B, and C of chiauranib and a preparation method thereof are disclosed in CN201610856945.2, which is incorporated by reference in its entirety.

In a specific embodiment, a dose of the chiauranib or the derivative thereof is about 1-100 mg, preferably about 5-80 mg, more preferably about 10-50 mg, and the most preferably about 1 mg, 2 mg, 3 mg, 4 mg, 5 mg, 10 mg, 15 mg, 20 mg, 25 mg, 30 mg, 35 mg, 40 mg, 45 mg, 50 mg, 55 mg or 60 mg.

The immune checkpoint inhibitor includes a PD-1 inhibitor, a PD-L1 inhibitor, a CTLA-4 inhibitor, a TIM-3 inhibitor, a BTLA inhibitor, a VISTA inhibitor, or an LAG-3 inhibitor, preferably a PD-1 inhibitor or a PD-L1 inhibitor.

The term *"PD-1 inhibitor"* refers to a moiety that reduces, inhibits, blocks, eliminates or interferes with the activity or expression of PD-1, such as a compound, a nucleic acid, a polypeptide, and an antibody, including a variant, an isotype, a homolog (e.g., a mouse) of human PD-1, and an analog sharing at least one common epitope with PD-1. A PD-1 inhibitor includes a small molecule and a large molecule, such as a compound, a nucleic acid, a polypeptide, an antibody, a peptibody, a bispecific antibody, a miniantibody, a single-chain variable fragment (ScFv), and a fragment or variant thereof. Therefore, the PD-1 inhibitor used in the present invention refers to any moiety that antagonizes the activity or expression of PD-1. PD-1 inhibitor includes an exemplary component such as nivolumab, pembrolizumab, toripalimab, sintilimab, SHR-1210, BGB-A317, geptanolimab, zimberelimab (AB122), AK101, AK104, AK105, GLS-010, BAT1306, CS1003, PDR001, cemiplimab, and MEDI0680.

Nivolumab, sold under the brand name Opdivo, was jointly developed by Medarex and Ono. In 2009, Bristol Myers Squibb (BMS) acquired nivolumab and obtained the patent for the drug. Nivolumab is the first approved PD-1 monoclonal antibody in the world, which can be used for treating cancer such as melanoma, non-small cell lung cancer, renal cell carcinoma, and head and neck cancer. The combination of nivolumab and ipilimumab (Yervoy) targeting CTLA-4 is the first approved immunotherapy combination in the world, which can be used for treating metastatic melanoma at a dose of 3 mg/kg.

Pembrolizumab, sold under the brand name of Keytruda, is an antibody product of Merck. After it was launched in US in 2014, its sales ranked second in the PD-1 antibody market. It is a strong competitor of nivolumab of Bristol Myers Squibb (BMS). The first indication approved of Pembrolizumab is melanoma, the second is in combination with chemotherapy for the first-line treatment for non-small cell lung adenocarcinoma, and the third is used alone as the first-line treatment for lung cancer (adenocarcinoma and squamous carcinoma) at a dose of pembrolizumab of 2 mg/kg.

Toripalimab, sold under the brand name of *Tuoyi,* is the first launched PD-1 inhibitor that is produced in China. It was independently developed by Junshi Biosciences. It was approved in 2018 for treating locally advanced or metastatic melanoma after failure of previous standard therapy. Junshi Biosciences was preparing to submit the marketing application of toripalimab for treating nasopharyngeal carcinoma and urothelial carcinoma.

Sintilimab, sold under the brand name of Tyvyt, was jointly developed by Innovent Biologics and Eli Lilly in China. As a PD-1 monoclonal antibody, it can bind to PD-1 on the surface of a T cell to block the binding of PD-1 to a ligand PD-L1, so that the T cell and autoimmune response can play a normal role, and then destroy tumor cells. Sintilimab was approved in China at the end of 2018 and listed in Chinese Society of Clinical Oncology (CSCO) diagnosis and treatment guidelines for lymphoma 2019. It is recommended for the treatment of relapsed/refractory classical Hodgkin lymphoma (R/R-cHL).

SHR-1210, *i.e.* camrelizumab, sold under the brand name of *AiRuiKa,* is a PD-1 inhibitor independently developed by Jiangsu HengRui Medicine Co., Ltd. The preclinical study on camrelizumab was completed in January 2015. An international multi-center phase III clinical trial for combined use of camrelizumab and apatinib mesylate as the first-line treatment for liver cancer was carried out simultaneously in the U.S., Europe, and China under the approval of the U.S. Food and Drug Administration (FDA) in January 2019. On May 29, 2019, the China National Medical Products Administration officially approved the marketing of *AiRuiKa.*

BGB-A317, *i.e.* Tislelizumab, sold under the brand name of *Baizean,* is a human monoclonal antibody developed by BeiGene. Tislelizumab may restore the ability of CTL to kill cancer cells by binding to PD-1 without activating a receptor, so as to prevent the binding of PD-L1 to PD-1. On December 27, 2019, the marketing application (CXSS1800019) of tislelizumab injection was approved by the China National Medical Products Administration for treating relapsed/refractory classical Hodgkin lymphoma (rr-cHL).

Other PD-1 inhibitors include AK101, AK104, and AK105 of Lepu Medical, geptanolimab of Genor Biopharma, GLS-010 of Gloria Pharmaceuticals, BAT1306 of Bio-Thera, CS1003 of Cstone Pharmaceuticals, PDR001 of Novartis, cemiplimab of Regeneron/Sanofi, MEDI0680 of AstraZeneca, zimberelimab (AB122) of Arcus, and the like.

The term "*PD-L1 inhibitor"* refers to a moiety that reduces, inhibits, blocks, eliminates or interferes with the activity of PD-L1, the binding of PD-L1 to its receptor PD-1, or the expression of PD-L1, such as a compound, a nucleic acid, a polypeptide, and an antibody, including a variant, an isotype, a homolog *(e.g.,* a mouse) of human PD-L1, and an analog sharing at least one epitope with PD-L1. PD-L1 inhibitor includes a small molecule and a large molecule, such as a small molecule compound, a nucleic acid, a polypeptide, an antibody, a peptibody, a bispecific antibody, a miniantibody, a single chain variable fragment (ScFv), and a fragment or variant thereof. Therefore, the PD-L1 inhibitor used in the present invention refers to any moiety that antagonizes the activity of PD-L1, the binding of PD-L1 to PD-1, and the expression of PD-L1. PD-L1 inhibitor includes an exemplary component such as Tecentriq (atezolizumab), Imfinzi (durvalumab), Bavencio (avelumab), CS1001, TQB2450, SHR1316, lazertinib, bintrafuspalfa, envafolimab (KN035), CA-170, CX-072, BGB-A333, BMS-936559, GEN-1046, KL-A167, and IO-103.

Tecentriq, *i.e*. Atezolizumab, is a PD-L1 immunosuppressant developed by Roche. It can bind to PD-L1 on tumor cells and tumor infiltrating immune cells to block the interaction of PD-L1 and PD-1 and B7-1 receptors, so as to reactivate T cells to kill cancer cells. In May 2020, Tecentriq (atezolizumab) was approved by the U.S. FDA for a new indication, that is, a first-line (initial) monotherapy of adult patients with metastatic non-small cell lung cancer (NSCLC), and specifically a detection method approved by FDA for determining advanced non-squamous and squamous non-small cell lung cancer (NSCLC) adult patients without EGFR or ALK mutations whose tumors have high PD-L1 expression (PD-L1 stained ≥ 50% of tumor cells [TC ≥ 50%] or PD-L1 stained tumor-infiltrating immune cells [IC] covering ≥ 10% of the tumor area [IC ≥ 10%]).

Durvalumab, sold under the brand name of Imfinzi, was developed by AstraZeneca. As a human monoclonal antibody directly targeting PD-L1, it can block the interaction between PD-L1 and PD-1 and CD80 on T cells to resist immune evasion means of tumors and induce immune responses. In 2020, the detailed results of the latest analysis of the phase III CASPIAN trial in lung cancer announced by AstraZeneca showed that the first-line treatment combining anti-PD-L1 therapy Imfinzi with stand of care (SoC) platinum-containing chemotherapy (etoposide + cisplatin or carboplatin) has a sustained clinically significant overall survival (OS) benefit for adult patients with extensive-stage small cell lung cancer (ES-SCLC).

Avelumab, sold under the brand name of Bavencio, is a PD-L1 antibody drug jointly developed by Pfizer and Merck KGaA. In May 2020, Pfizer and Merck disclosed positive results from interim analysis of JAVELIN Bladder 100, a phase 3 clinical trial in which maintenance treatment with anti-PD-L1 drug Bavencio (avelumab) plus best supportive care (BSC) in patients with advanced urothelium carcinoma (UC) after receiving platinum-based chemotherapy was compared to treatment with BSC alone. The median follow-up of patients in the Avelumab + BSC treatment group or the BSC treatment group was 19.6 months and 19.2 months, respectively. Among all the enrolled patients, 358 cases tested positive for the tumor biomarker PD-L1. The results show that compared with use of BSC alone, avelumab + BSC significantly increases overall survival (OS) (hazard ratio [HR] 0.69; 95% CI 0.56, 0.86; 1-sided p=0.0005) of the patients; and median OS of the avelumab + BSC group and the BSC group is 21.4 months and 14.3 months, respectively, which indicates that OS of the patients is increased by more than 7 months.

Envafolimab (KN035) is the first nanobody produced in China and targets PD-L1, which was developed by Alphamab Oncology. KN035 was authorized to 3D Medicines in March 2016 and applied for clinical application in May of the same year. The structure of KN035 is a PD-L1 nanobody-Fc fusion protein, Fc adopts IgG1 subtype, and Fc effector effects, such as ADCC, are removed by three point mutations of C221S, D265A, and P331G. A KN035 preparation is a room temperature stable subcutaneous injection, which is easy to store and use. KN035 is a PD-L1 single-domain antibody Fc fusion protein. Based on the unique design, it demonstrates advantages in safety, convenience, and compliance, and can be used for patients who are not suitable for intravenous infusion with a lower medical cost. At present, KN035 is being evaluated in clinical trials for multiple cancer indications in China, the United States, and Japan, and the research for some indications have entered phase III clinical trials.

CS1001, TQB2450, and SHR1316 are respectively PD-L1 inhibitors being developed by Cstone Pharmaceuticals, Chia Tai Tianqing, and Hengrui Pharmaceuticals.

In specific embodiments, a dose of the immune checkpoint inhibitor is about 1-1000 mg, preferably about 10-800 mg, more preferably 20-500 mg, and the most preferably about 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 300 mg, 400 mg, or 500 mg.

Wherein, the tumor includes colon cancer, liver cancer, lung cancer, stomach cancer, intestinal cancer, breast cancer, cervical cancer, rectal cancer, pancreatic cancer, brain cancer, skin cancer, oral cancer, prostate cancer, bone cancer, kidney cancer, ovarian cancer, bladder cancer, fallopian tube tumor, peritoneal tumor, melanoma, glioma, glioblastoma, hepatocellular carcinoma, head and neck tumor, leukemia, lymphoma, and myeloma.

The present invention also provides a pharmaceutical composition including the foregoing combination.

The pharmaceutical composition of the present invention includes a pharmaceutical composition administered to a patient by any route, such as oral, mucosal (nasal, inhalational, pulmonary, sublingual, vaginal or rectal), parenteral (subcutaneous, intravenous, rapid injection, intramuscular or intra-arterial), and percutaneous.

A dosage form of the pharmaceutical composition of the present invention includes: solid preparations such as tablets, granules, and capsules, liquid dosage forms suitable for parenteral administration to patients, and liquid dosage forms suitable for parenteral administration to patients.

The pharmaceutical composition of the present invention includes one or more excipients, and suitable excipients are well known to those skilled in the field of medicines.

The present invention also provides a kit, including the foregoing combination.

The kit of the present invention may include the foregoing combination with the same or different preparations. Components in the foregoing combination in the kit may be provided in separate independent vessels or placed in the same vessel.

In a specific embodiment, the chiauranib or the derivative thereof in the kit is prepared into solid preparations suitable for oral administration, such as tablets, granules, and capsules. The immune checkpoint inhibitor may be prepared into a form such as lyophilized powder and a solution for parenteral administration.

In a specific embodiment, the chiauranib or the derivative thereof and the immune checkpoint inhibitor are unit preparations with the same or different specifications.

In a specific embodiment, the chiauranib or the derivative thereof and the immune checkpoint inhibitor are placed in the same vessel or respectively placed in different vessels.

In a specific embodiment, the chiauranib or the derivative thereof is in a dosage form for gastrointestinal administration, and is preferably an oral preparation; the immune checkpoint inhibitor is in a dosage form for parenteral administration, and is preferably an injectable preparation.

The kit also includes instructions for use or other information.

The present invention also provides a method for treating a tumor, which includes the following steps: administering a therapeutically effective amount of the foregoing pharmaceutical composition or kit to a tumor patient in need. The method for treating a tumor provided in the present invention is a combined administration regimen.

The tumor includes colon cancer, liver cancer, lung cancer, stomach cancer, intestinal cancer, breast cancer, cervical cancer, rectal cancer, pancreatic cancer, brain cancer, skin cancer, oral cancer, prostate cancer, bone cancer, kidney cancer, ovarian cancer, bladder cancer, fallopian tube tumor, peritoneal tumor, melanoma, glioma, glioblastoma, hepatocellular carcinoma, head and neck tumor, leukemia, lymphoma, and myeloma.

The term *"combined administration"* refers to administration of two or more pharmaceutical ingredients. The timing of combined administration depends on a combination to be administered. Combined administration is intended to include administration of a drug alone, or simultaneous or sequential administration of a combination. The components of the present invention may be administered alone or may be administered to a patient in combination. The components of the present invention may be used in combination with each other, or may also be used in combination with other active reagents known to be useful in the treatment of cancer.

The term *"administration"* refers to delivery of the combination to a subject by a route such as oral, mucosal, topical, suppository, intravenous, parenteral, intraperitoneal, intramuscular, intralesional, intrathecal, intranasal or subcutaneous administration. Parenteral administration includes intravenous, intramuscular, intra-arteriolar, intradermal, subcutaneous, intraperitoneal, intraventricular, and intracranial administration. Administration typically occurs after the onset of a disease, disorder or condition or a symptom thereof, but may occur prior to the onset of a disease, disorder or condition or a symptom thereof in some cases.

Preferably, the pharmaceutical composition or kit is administered to the tumor patient as a first-line treatment.

The foregoing pharmaceutical composition or kit may also be administered to the tumor patient as a second-line, third-line, fourth-line, fifth-line or sixth line treatment.

Preferably, the patient has not been treated or has undergone at least one antitumor treatment.

The antitumor treatment includes surgery, chemotherapy, radiotherapy, targeted therapy, and immunotherapy, or a combination thereof.

In the method, the chiauranib and the derivative thereof and the immune checkpoint inhibitor are administered simultaneously, separately or sequentially.

The present invention has the following beneficial effects:
(1) Compared with a solvent control group, proportions of spleen lymphocyte subsets CD4 and CD8 in mice in a chiauranib administration group and a PD-1 antibody administration group are increased to a certain extent, which are respectively increased from average proportions of 5.8% (CD4) and 3.5% (CD8) of the solvent control group to 9.9% and 12% (CD4), and 6.1% and 6% (CD8). However, proportions of spleen CD4 and CD8 subsets of a chiauranib and PD-1 antibody combined administration group are significantly increased, respectively 15.8% and 9.3%.
(2) Compared with the solvent control group, relative tumor inhibition rates of the chiauranib administration group and the PD-1 antibody administration are respectively 39% and 32%, and the combined use of the two drugs significantly increases the tumor inhibition rate to 70%. The foregoing results show that the combination of chiauranib and the PD-1 antibody has good immunopotentiation and synergistic antitumor activity in tumor-bearing mice.
(3) Compared with the solvent control group, relative tumor inhibition rates of the chiauranib administration group and the murine PD-1 antibody administration are respectively 26% and 39%, and the combined use of the two drugs significantly increases the relative tumor inhibition rate to 51%. The results show that the combination of chiauranib and the immune checkpoint inhibitor PD-1 antibody has good synergistic antitumor activity in tumor mouse models.
(4) The chiauranib (2.5 mg/kg) group, the reference PD-L1 antibody (1 mg/kg and 3 mg/kg) groups, the chiauranib (2.5 mg/kg) and reference PD-L1 antibody (1 mg/kg) group, and the chiauranib (2.5 mg/kg) and reference PD-L1 antibody (3 mg/kg) group at test doses have significant inhibitory effects on the growth of subcutaneously transplanted tumor MC38-hPD-L1, do not produce toxic and side effects on animals when they exert effects, and have good safety.

The foregoing experimental data shows that the combination of chiauranib or a derivative thereof and the immune checkpoint inhibitor has a synergistic effect, and is suitable for treatment of cancer.

### BRIEF DESCRIPTION OF THE DRAWINGS

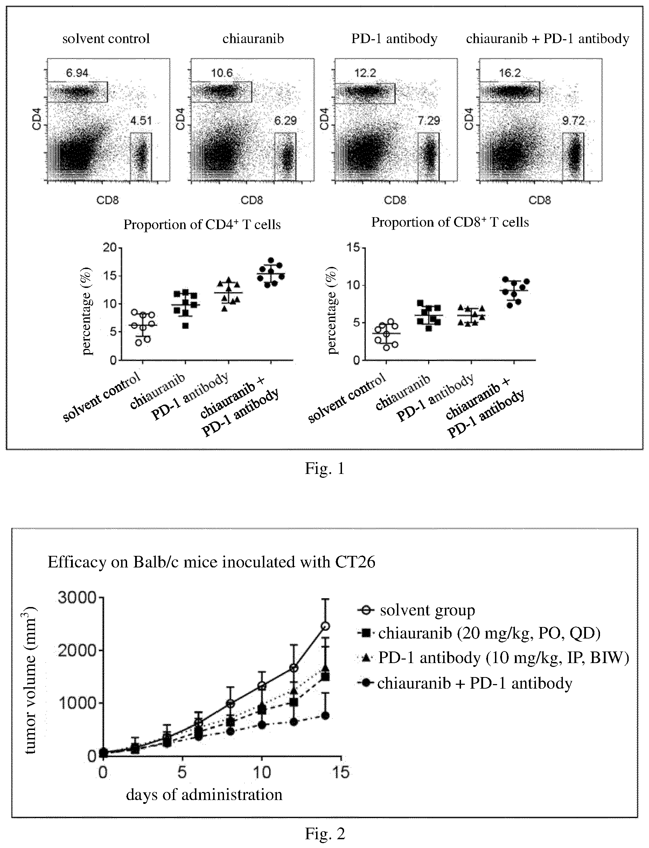

Fig. 1 shows effects of administration of chiauranib alone, a PD-1 antibody alone, and a combination of chiauranib and the PD-1 antibody on proportions of splenic lymphocyte subsets in tumor-bearing mice.
Fig. 2 shows pharmaceutical effects of administration of chiauranib alone, a PD-1 antibody alone, and a combination of chiauranib and the PD-1 antibody on mice inoculated with a colonic cancer cell line CT-26.

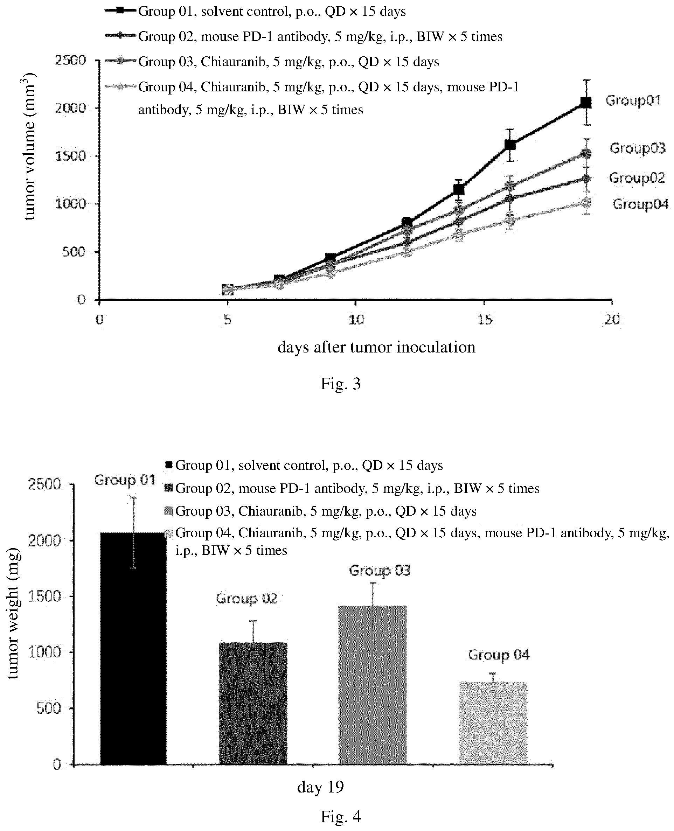

Fig. 3 shows growth curves of the tumor volume of the mice in the groups affected by the pharmaceutical effects of administration of chiauranib alone, a PD-1 antibody alone, and a combination of chiauranib and the PD-1 antibody on mice inoculated with a liver cancer cell line H22, where data is expressed as "mean ± standard error".
Fig. 4 shows experimental endpoint tumor weights of the mice in the groups affected by the pharmaceutical effects of administration of chiauranib alone, a PD-1 antibody alone, and a combination of chiauranib and the PD-1 antibody on mice inoculated with a liver cancer cell line H22, where data is expressed as "mean ± standard error".

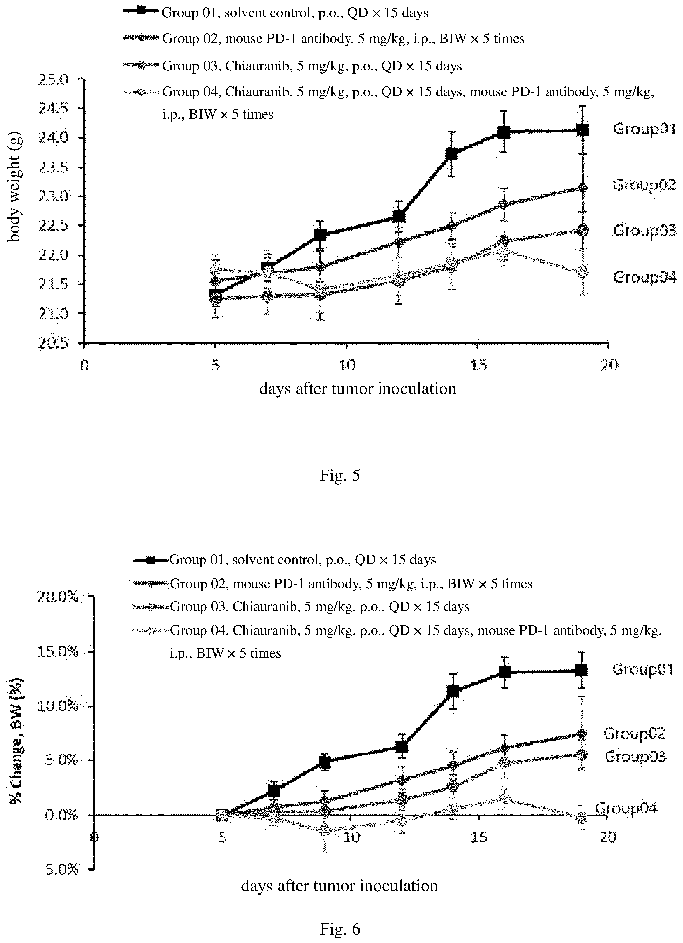

Fig. 5 shows body weight change curves with treatment time of the groups affected by the pharmaceutical effects of administration of chiauranib alone, a PD-1 antibody alone, and a combination of chiauranib and the PD-1 antibody on mice inoculated with a liver cancer cell line H22, where data is expressed as "mean ± standard error".
Fig. 6 shows body weight change rate curves with treatment time of the groups affected by the pharmaceutical effects of administration of chiauranib alone, a PD-1 antibody alone, and a combination of chiauranib and the PD-1 antibody on mice inoculated with a liver cancer cell line H22, where data is expressed as "mean ± standard error".

### DETAILED DESCRIPTION

The present invention discloses the use of chiauranib in combination with an immune checkpoint inhibitor in antitumor therapy. Those skilled in the art may appropriately improve process parameters to implement the present invention by referring to the content of the present invention. It should be particularly pointed out that all similar replacements and modifications are obvious to those skilled in the art, and they are all considered to be included in the present invention. The use and pharmaceutical composition of the present invention have been described through preferred embodiments, and relevant personnel can obviously make modifications or appropriate changes and combinations to the use and pharmaceutical composition described herein without departing from the content, spirit, and scope of the present invention and, to implement and apply the technology of the present invention.

### Example I Efficacy on tumor Balb/c mouse models inoculated with a colonic cancer cell line CT-26

### Experimental materials:

A mouse colonic cancer cell line CT-26 was purchased from the Cell Bank, Shanghai Institutes for Biological Sciences, Chinese Academy of Sciences, and conventionally cultured at 37°C under 5% CO₂ in DMEM (Gibco) containing 10% fetal bovine serum (FBS; Gibco) and 1% penicillin-streptomycin (HyClone); and trypsin was purchased from Gibco. Chiauranib with a purify greater than 99% was synthesized by Shenzhen Chipscreen Biosciences Co., Ltd. Antimouse PD-1 antibodies were all purchased from Bio X cell. Fluorescein labelled anti-CD4 and CD8 antibodies for detecting lymphocyte subsets in mice were purchased from eBiosciences. Normal 6-8-week-old Balb/c mice were purchased from Guangdong Medical Laboratory Animal Center.

### Experimental method:

A large number of CT-26 cells were cultured and kept in a logarithmic growth state. After the number of cells reached a required level, the cells were digested and collected by trypsin, thoroughly washed twice with a large amount of PBS to remove trypsin and serum components, centrifuged at room temperature at 800 rpm for 10 min, and a supernatant was discarded. The cells were resuspended in DMEM without FBS, and the cell concentration was adjusted to 5×10⁶ cells/mL.

Under sterile conditions, the cell suspension was injected subcutaneously into the back of Balb/c mice at 100 µL/injection, one injection per mouse. A 1 mL disposable medical syringe was used for injection to ensure that injection sites and directions of the mice were basically the same. 8 days after cell inoculation, tumors grew to an average volume of about 100 mm³, and the tumor-bearing mice were randomly divided into four groups (9 mice per group), that is, a solvent control group, a chiauranib group (20 mg/kg, intragastric administration, once a day), a PD-1 antibody group (10 mg/kg, intraperitoneal injection, twice a week), and a chiauranib and PD-1 antibody combined administration group, marked, raised in separate cages, administered daily in groups, and observed for tumor formation. The longest diameter (length) and the widest diameter (width) perpendicular to the longest diameter of the tumor was measured by using a vernier caliper every 2 days, and the tumor volume was calculated according to a formula: TS = length × (width)² / 2, and recorded. The mice were subjected to intragastric administration once a day regularly, and after the last administration on the 14th day, the mice were sacrificed to take the spleens and make a single cell suspension. 1×10⁶ spleen cells were taken and stained with fluorescein labelled anti-CD4 and CD8 antibodies (1: 200) at 4°C for 30 min. After being washed, the cells were resuspended in PBS, and detected by using a flow cytometer FACSCanto II. A relative tumor inhibition rate of each administration group is equal to: (average tumor volume of solvent control group - average tumor volume of administration group) / average tumor volume of solvent control group × 100%.

### Experimental results:

As shown in Fig. 1, compared with the solvent control group, proportions of spleen lymphocyte subsets CD4 and CD8 in the mice in the chiauranib administration group and the PD-1 antibody administration group are increased to a certain extent, which are respectively increased from average proportions of 5.8% (CD4) and 3.5% (CD8) of the solvent control group to 9.9% and 12% (CD4), and 6.1% and 6% (CD8). However, proportions of spleen CD4 and CD8 subsets of the chiauranib and PD-1 antibody combined administration group are significantly increased, which are respectively 15.8% and 9.3%.

As shown in Fig. 2, compared with the solvent control group, relative tumor inhibition rates of the chiauranib administration group and the PD-1 antibody administration group are respectively 39% and 32%, and the combined use of the two drugs significantly increases the relative tumor inhibition rate to 70%. The foregoing results show that the combination of chiauranib and the PD-1 antibody has good immunopotentiation and synergistic antitumor activity in tumor-bearing mice.

### Example II Efficacy on female tumor BALB/c mouse models subcutaneously transplanted with a mouse liver cancer H22 cell line

### Experimental animals:

Female 7-9-week-old (at the time of inoculation of tumor cells) BALB/c mice weighing about 22 g were purchased from Shanghai Lingchang Biotechnology Co., Ltd, with the production license No. SCXK (Hu) 2018-0003 and the animal certificate No. 20180003015834. Breeding environment: SPF level.

All the experimental animals were kept in independent ventilated boxes with constant temperature and humidity, the temperature of the feeding room was 20-26°C, the humidity was 40-70%, air was changed at a rate of 10-20 times/h, and day and night (light and dark) alternate time was 12 h/ 12 h. Cobalt-60 radiation sterilized mouse complete pellet feed was continuously supplied in an unlimited free intake manner, and tap water (used after high-pressure steam sterilization) was supplied uninterruptedly from drinking bottles in a free intake manner. The mouse feeding boxes were polysulfone mouse boxes with a specification of 325 mm × 210 mm × 180 mm, which were used after high-pressure sterilization. Bedding was autoclaved corn cobs, and 5 experimental animals were placed in each box, and earmarked.

### Experimental materials:

Chiauranib was provided by Shenzhen Chipscreen Biosciences Co., Ltd., with the batch No. 20101008, which was white powder and sealed and stored at room temperature. Mouse PD-1 antibody: batch No. 0920L765, packing specification: 30 mg, 10.1 mg/mL, was provided by Crown Bioscience, Inc. (Taicang), which was a colorless solution and sealed and stored at 4°C. CMC-Na: supplier: SIGMA-ALDRICH; batch No. SLBK436V. Tween 80: supplier: SIGMA-ALDRICH; batch No. WXBC7734V. PBS: supplier: Hyclone; batch No. AF29477519.

### Experimental method:

Mouse liver cancer H22 cells (purchased from CCTCC; cell No. TC-00011) were cultured in an RPMI-1640 medium (purchased from Gibco) containing 10% fetal bovine serum. H22 cells in exponential growth phase were collected, resuspended in PBS to an appropriate concentration, and subcutaneously inoculated into mice. Female mice were subcutaneously inoculated with 1×10⁶ H22 cells on the right front side. After tumors grew to an average volume of 104 mm³, the mice were randomly grouped (see Table 1) according to the tumor size, administration was carried out on the day of grouping, and the day of inoculation of tumor cells was defined as day 0.

**Table 1 Dosage regimen**

| Group | Number of animals | Administration group | Dose (mg/kg) | Administration method | Administration Period |
|---|---|---|---|---|---|
| 1 | 10 | **Solvent control** (0.2% CMC-Na + 0.1% Tween 80, respectively weigh 0.6 g of CMC-Na powder and 0.3 g of Tween 80, add 300 mL of purified water, heat and stir until completely dissolved, and use after sterilization) | -- | Orally intragastric administration | QD × 15 days |
| 2 | 10 | **Mouse PD-1 antibody** (dilute in PBS to 0.5 mg/mL) | 5 | Intraperitoneal injection | BIW × 5 times |
| 3 | 10 | **Chiauranib** (9 mg of chiauranib powder, add 18 mL of solvent containing 0.2% CMC-Na + 0.1% Tween 80, and ultrasonically shake for 5 min until uniformly disperse) | 5 | Orally intragastric administration | QD × 15 days |
| 4 | 10 | **Chiauranib** | 5 | Orally intragastric administration | QD × 15 days |
| | | **Mouse PD-1 antibody** | 5 | Intraperitoneal injection | BIW × 5 times |

| | | | | | |
|---|---|---|---|---|---|
| Note: 1. Administration volume is 10 µL/g; 2. First administer chiauranib, and then administer the mouse PD-1 antibody, no specific dosing interval. QD means once a day, BIW means twice a week. | | | | | |

### Experimental results:

On the 19th day of the experiment (day 15 of administration in groups), an average tumor volume of the solvent control group was measured to be 2060.99 mm³, the experiment was completed, photos of the tumor-bearing mice were taken, the tumors were taken out and weighed, and photos of the tumors were taken. A formula for calculating tumor volume: tumor volume (mm³)=1/2×(*a*×*b*²) (where, *a* represents a long diameter, and *b* represents a short diameter).

At the end of the experiment (on the 19th day of the experiment, and day 15 of administration), an average tumor volume of the mice in the solvent control group was 2060.99 mm³. At the end of the experiment, an average tumor volume of the mouse PD-1 antibody (5 mg/kg) treatment group was 1266.93 mm³, which had a statistically significant difference (p=0.0242) compared to the control group, and TGI was 39%. An average tumor volume of the chiauranib (5 mg/kg) treatment group was 1527.40 mm³, which had no statistically significant difference (p=0.173) compared to the control group, and TGI was 26%. An average tumor volume of the chiauranib (5 mg/kg) and mouse PD-1 antibody (5 mg/kg) combined treatment group was 1013.80 mm³, which had a statistically significant difference (p=0.00126) compared to the control group, and TGI was 51%. Tumor samples were collected at the end of the experiment and weighed, and results were the same as those of tumor volume measurement. Tumor growth of the treatment groups and the control group is shown in Table 2, Fig. 3, and Fig. 4.

Compared with the solvent control group, relative tumor inhibition rates of the chiauranib administration group and the mouse PD-1 antibody administration group are respectively 26% and 39%, and the combined use of the two drugs significantly increases the relative tumor inhibition rate to 51%. The results show that the combination of chiauranib and the immune checkpoint inhibitor anti-PD-1 antibody has good synergistic antitumor activity in tumor mouse models.

**Table 2 Efficacy on models of subcutaneously transplanted mouse liver cancer H22 in groups**

| Group | Tumor volume (mm3) Day 5^{a} | Tumor volume (mm3) Day 19^{a} | RTV | T/C (%) Day 19^{b} | TGI (%) Day 19^{b} | P value^{c} Day 19 |
|---|---|---|---|---|---|---|
| Group 1, solvent control | 103.98±3.51 | 2060.99±231.86 | 19.51 | - | - | - |
| Group 2, mouse PD-1 antibody; 5 mg/kg | 104.00±3.14 | 1266.93±215.51 | 12.36 | 61 | 39 | 0.0242 |
| Group 3, chiauranib; 5 mg/kg | 104.09±2.73 | 1527.40±145.56 | 14.51 | 74 | 26 | 0.173 |
| Group 4, chiauranib; 5 mg/kg in combination with mouse PD-1 antibody; 5 mg/kg | 104.06±2.65 | 1013.81±120.36 | 9.75 | 49 | 51 | 0.00126 |

| | | | | | | |
|---|---|---|---|---|---|---|
| Note: a. Data is expressed as "mean ± standard error"; b. T/C%=T_{RTV}/C_{RTV}×100%; TGI%=(1-T/C)×100% (T_{RTV}: mean RTV of treatment group; C_{RTV}: mean RTV of solvent control group; RTV = Vₜ/V₀, V₀ is tumor volume of an animal when grouped, and Vₜ is tumor volume of the animal after treatment); c. Relative tumor volume significant differences between the control group and the treatment groups are detected by one-way ANOVA and TukeyHSD. P less than 0.05 is considered to have a significant difference. | | | | | | |

The body weight was measured 3 times a week. Body weight changes of the treatment groups and the control group are shown in Fig. 5, Fig. 6, and Table 3. It can be seen from Fig. 5, Fig. 6, and Table 3 that one mouse in the mouse PD-1 antibody (5 mg/kg) treatment group died, and other mice in the groups had no significant body weight loss during the experiment, did not show obvious drug toxicity, and well tolerated during the treatment.

**Table 3 Body weight changes of models of subcutaneously transplanted mouse liver cancer H22 in groups at the end of experiment**

| Experimental group | Number of animals at the beginning/end of experiment | Average body weight (g)^{a} | | Body weight change rate (%) |
|---|---|---|---|---|
| | | Day 5 | Day 19 | Day 19 |
| Group 1, solvent control | 10/10 | 21.3±0.2 | 24.1±0.4 | 13.24% |
| Group 2, mouse PD-1 antibody; 5 mg/kg | 10/9 | 21.6±0.4 | 23.2±0.8 | 7.46% |
| Group 3, chiauranib; 5 mg/kg | 10/10 | 21.3±0.3 | 22.4±0.3 | 5.59% |
| Group 4, chiauranib; 5 mg/kg in combination with mouse PD-1 antibody; 5 mg/kg | 10/10 | 21.8±0.3 | 21.7±0.4 | -0.25% |

| | | | | |
|---|---|---|---|---|
| Note: a. Data is expressed as "mean ± standard error". | | | | |

### Example III Efficacy on animal models of B-hPD-1/hPD-L1 humanized mouse MC38-hPD-L1 colon cancer

### Experimental materials:

Mouse colon cancer MC38 cells were purchased from Shunran Biology, cultured in an incubator under 5% CO₂ at 37°C, and a medium was Dulbecco's Modified Eagle's Medium containing 10% inactivated fetal bovine serum. Biocytogen carried out genetic modification on the MC38 cells to enable the MC38 cells to overexpress human PD-L1, and knocked out mouse PD-L1, and the cells were named MC38-hPD-L1 cells. A reference PD-L1 antibody used in the experiment was constructed and produced by the in-house laboratory according to a sequence of the marketed antibody drug MPDL3280A.

### Experimental method:

### Cell culture and tumor inoculation

A certain number of cells were cultured and collected, the MC38-hPD-L1 cells resuspended in PBS were subcutaneously inoculated into B-hPD-1/hPD-L1 humanized mice on the right side at a concentration of 5×10⁵ cells/0.1 mL and a dose of 0.1 mL/mouse. After an average tumor volume reached about 104 mm³, appropriate mice were selected according to tumor volume and body weight, and uniformly divided into 6 experimental groups, 8 mice per group, and administration was carried out on the day of grouping. A specific dosage regimen is shown in Table 4.

**Table 4 Dosage regimen**

| Group | Test product | Number of animals (individuals) | Dose (mg/kg)^{a} | Administration route | Administration frequency^{b} | Administration period |
|---|---|---|---|---|---|---|
| G1 | Control solution (0.2% CMC-Na and 0.1% Tween 80 solution) | 8 | 200 µL | p.o. | QD | 21 |
| G2 | Chiauranib | 8 | 2.5 | p.o. | QD | 21 |
| G3 | Reference PD-L1 antibody | 8 | 1 | i.p. | Q3D | 7 |
| G4 | Reference PD-L1 antibody | 8 | 3 | i.p. | Q3D | 7 |
| G5 | Chiauranib | 8 | 2.5 | p.o. | QD | 21 |
| | Reference PD-L1 antibody | | 1 | i.p. | Q3D | 7 |
| G6 | Chiauranib | 8 | 2.5 | p.o. | QD | 21 |
| | Reference PD-L1 antibody | | 3 | i.p. | Q3D | 7 |

| | | | | | | |
|---|---|---|---|---|---|---|
| Note: a: An administration volume is calculated according to the body weight of the experimental animal at 10 µL/g. b: QD indicates administration once a day, Q3D indicates administration once every three days. | | | | | | |

### Experimental results:

### (1) Responses and body weight changes of experimental animals after administration

During the experiment, the experimental animals were in a good state of activity and eating during the administration, and gained body weight to a certain extent, indicating that the animals tolerated the test products well. Body weight changes of all the animals are shown in Table 5.

**Table 5 Effects of test products on body weight of B-hPD-1/hPD-L1 humanized mice transplanted with MC38-hPD-L1 colon cancer cells**

| Group | Test product | Body weight (g)^{a} | | | Body weight change after 38 days of administration (g) |
|---|---|---|---|---|---|
| | | Before administration | Day 27 of administration in groups | P^{b} | |
| G1 | Control solution (0.2% CMC-Na and 0.1% Tween 80 solution) | 21.3±0.4 | 25.0±0.4 | - | +3.7 |
| G2 | Chiauranib | 21.1±0.3 | 24.2±0.6 | 0.294 | +3.1 |
| G3 | Reference PD-L1 antibody (1 mg/kg) | 21.6±0.3 | 25.4±0.4 | 0.549 | +3.8 |
| G4 | Reference PD-L1 antibody (3 mg/kg) | 21.3±0.5 | 25.0±0.5 | 0.940 | +3.7 |
| G5 | Chiauranib | 21.1±0.3 | 24.1±0.4 | 0.132 | +3.0 |
| | Reference PD-L1 antibody (1 mg/kg) | | | | |
| G6 | Chiauranib | 21.2±0.3 | 23.8±0.4 | 0.056 | +2.6 |
| | Reference PD-L1 antibody (3 mg/kg) | | | | |

| | | | | | |
|---|---|---|---|---|---|
| Note: a: mean ± standard error; b: statistical comparison of the body weight of the administration group and the body weight of the control group on day 27 of administration in groups, T-test analysis. | | | | | |

### (2) Tumor volume inhibition results

Results were recorded, tumor inhibition rates (TGITV) were calculated, and statistical analysis between groups was carried out according to tumor volume. Results are shown in Table 6 and Table 7.

At the end of the experiment (that is, day 27 of administration in groups), compared with the control group, the chiauranib (2.5 mg/kg) group, the reference PD-L1 antibody (1 mg/kg and 3 mg/kg) groups, the chiauranib (2.5 mg/kg) and reference PD-L1 antibody (1 mg/kg) combined administration group, and the chiauranib (2.5 mg/kg) and reference PD-L1 antibody (3 mg/kg) combined administration group at test doses have significant inhibitory effects (P<0.05) on the growth of MC38-hPD-L1 subcutaneously transplanted tumors.

**Table 6 Effects of test products on tumor volume of B-hPD-1/hPD-L1 humanized mice transplanted with MC38-hPD-L1 colon cancer cells**

| Group | Test product | Tumor volume (mm³)^{a} | | | P^{b} |
|---|---|---|---|---|---|
| | | Before administration | Day 27 of administration in groups | TGI_{TV} (%) | |
| G1 | Control solution (0.2% CMC-Na and 0.1% Tween 80 solution) | 104±3 | 2322±261 | - | - |
| G2 | Chiauranib | 104±4 | 1021±158 | 58.6 | **<0.001 |
| G3 | Reference PD-L1 antibody (1 mg/kg) | 104±4 | 1469±144 | 38.5 | *0.013 |
| G4 | Reference PD-L1 antibody (3 mg/kg) | 104±3 | 1207±195 | 50.3 | **0.004 |
| G5 | Chiauranib | 104±4 | 1076±155 | 56.2 | **0.001 |
| | Reference PD-L1 antibody (1 mg/kg) | | | | |
| G6 | Chiauranib | 104±3 | 803±83 | 68.5 | **<0.001 |
| | Reference PD-L1 antibody (3 mg/kg) | | | | |

| | | | | | |
|---|---|---|---|---|---|
| Note: a: mean ± standard error; b: statistical comparison of tumor volume of the administration group and tumor volume of the control group on day 27 of administration in groups, T-test analysis, *P<0.05, **P<0.01. | | | | | |

**Table 7 Comparison of effects of different administration combinations on tumor volume of B-hPD-1/hPD-L1 humanized mice transplanted with MC38-hPD-L1 colon cancer cells**

| Group | G2 | G3 | G4 | G5 | G6 |
|---|---|---|---|---|---|
| G1 | **0.0008 | *0.0126 | **0.0041 | **0.0011 | **0.0001 |
| G2 | | 0.0549 | 0.4702 | 0.8082 | 0.2423 |
| G3 | | | 0.2982 | 0.0848 | **0.0013 |
| G4 | | | | 0.6064 | 0.0771 |
| G5 | | | | | 0.1438 |

| | | | | | |
|---|---|---|---|---|---|
| Note: statistical comparison of tumor volume of the administration group and tumor volume of the control group on day 27 of administration in groups, T-test analysis, *P<0.05, **P<0.01. | | | | | |

### (3) Tumor weight inhibition results

On day 27 of administration in groups, the whole experiment was completed. Tumor weight results of the groups are shown in Table 8.

Through data analysis, it can be seen that at the end of the experiment, compared with the control group, the chiauranib (2.5 mg/kg) group, the reference PD-L1 antibody (1 mg/kg and 3 mg/kg) groups, the chiauranib (2.5 mg/kg) and reference PD-L1 antibody (1 mg/kg) combined administration group, and the chiauranib (2.5 mg/kg) and reference PD-L1 antibody (3 mg/kg) combined administration group at test doses have significant inhibitory effects (P<0.05) on the growth of MC38-hPD-L1 subcutaneously transplanted tumors.

**Table 8 Inhibitory effects of test products on tumor weight of B-hPD-1/hPD-L1 humanized mice transplanted with MC38-hPD-L1 colon cancer cells**

| Group | Test product | Tumor weight (g)^{a} | TGI_{TW} (%) | P^{b} |
|---|---|---|---|---|
| G1 | Control solution (0.2% CMC-Na and 0.1% Tween 80 solution) | 2.185±0.282 | - | - |
| G2 | Chiauranib | 0.837±0.108 | 61.7 | **0.001 |
| G3 | Reference PD-L1 antibody (1 mg/kg) | 1.316±0.106 | 39.8 | *0.012 |
| G4 | Reference PD-L1 antibody (3 mg/kg) | 1.182±0.199 | 45.9 | *0.011 |
| G5 | Chiauranib | 0.897±0.127 | 59.0 | **0.001 |
| | Reference PD-L1 antibody (1 mg/kg) | | | |
| G6 | Chiauranib | 0.665±0.064 | 69.6 | **<0.001 |
| | Reference PD-L1 antibody (3 mg/kg) | | | |

| | | | | |
|---|---|---|---|---|
| Note: a: mean ± standard error. b: statistical comparison of tumor weight of the test drug group and the tumor weight of the control group at the end of experiment, T-test analysis, *P<0.05, and **P<0.01. | | | | |

### Experiment summaries:

In the experiment, the chiauranib (2.5 mg/kg) group, the reference PD-L1 antibody (1 mg/kg and 3 mg/kg) groups, the chiauranib (2.5 mg/kg) and reference PD-L1 antibody (1 mg/kg) combined administration group, and the chiauranib (2.5 mg/kg) and reference PD-L1 antibody (3 mg/kg) combined administration group at test doses have significant inhibitory effects on the growth of MC38-hPD-L1 subcutaneously transplanted tumors, do not produce toxic side effects to animals when playing a role, and have good safety.

The use of chiauranib in combination with an immune checkpoint inhibitor in antitumor therapy provided in the present invention has been described in details above. Herein, the specific examples are used to describe the principles and embodiments of the present invention. The description of the foregoing embodiments is only used to help understand the method and core idea thereof of the present invention, including the best mode, and also enables those skilled in the art to practice the present invention, including making and using any device or system, and implementing any combination method. It should be pointed out that those skilled in the art may further make some improvements and modifications to the present invention without departing from the principles of the present invention, and these improvements and modifications shall also fall within the scope of protection of the claims of the present invention. The patentable scope of the present invention is defined by the claims, and may include other embodiments that occur to those skilled in the art. Such other examples are intended to be within the scope of the claims if they have structural elements that do not differ from the literal expression of the claims, or if they include equivalent structural elements with insubstantial differences from the literal expression of the claims.

## Claims

1. Use of a combination of chiauranib or a derivative thereof and an immune checkpoint inhibitor in the preparation of a drug for treating a tumor.

2. The use according to claim 1, wherein the chiauranib derivative comprises a pharmaceutically acceptable salt thereof and non-solvated crystals A, B, and C thereof.

3. The use according to any one of claims 1 to 2, wherein a dose of the chiauranib or the derivative thereof is 1-100 mg, preferably 5-80 mg, and the most preferably 10-50 mg.

4. The use according to claim 1, wherein the immune checkpoint inhibitor comprises a PD-1 inhibitor, a PD-L1 inhibitor, a CTLA-4 inhibitor, a TIM-3 inhibitor, a BTLA inhibitor, a VISTA inhibitor, or an LAG-3 inhibitor, preferably a PD-1 inhibitor and a PD-L1 inhibitor.

5. The use according to claim 4, wherein the PD-1 inhibitor comprises nivolumab, pembrolizumab, toripalimab, sintilimab, SHR-1210, BGB-A317, geptanolimab, zimberelimab (AB122), AK101, AK104, AK105, GLS-010, BAT1306, CS1003, PDR001, cemiplimab, and MEDI0680.

6. The use according to claim 4, wherein the PD-L1 inhibitor comprises Tecentriq (atezolizumab), Imfinzi (durvalumab), Bavencio (avelumab), CS1001, TQB2450, SHR1316, lazertinib, bintrafuspalfa, envafolimab (KN035), CA-170, CX-072, BGB-A333, BMS-936559, GEN-1046, KL-A167, and 10-103.

7. The use according to any one of claims 4 to 6, wherein a dose of the immune checkpoint inhibitor is 1-1000 mg, preferably 10-800 mg, and most preferably 20-500 mg.

8. The use according to any one of claims 1 to 7, wherein the tumor comprises colon cancer, liver cancer, lung cancer, stomach cancer, intestinal cancer, breast cancer, cervical cancer, rectal cancer, pancreatic cancer, brain cancer, skin cancer, oral cancer, prostate cancer, bone cancer, kidney cancer, ovarian cancer, bladder cancer, fallopian tube tumor, peritoneal tumor, melanoma, glioma, glioblastoma, hepatocellular carcinoma, head and neck tumor, leukemia, lymphoma, and myeloma.

9. A pharmaceutical composition comprising the combination according to any one of claims 1 to 8.

10. A kit comprising the combination according to any one of claims 1 to 8.

11. The kit according to claim 10, wherein the chiauranib or the derivative thereof and the immune checkpoint inhibitor are unit preparations with the same or different specifications.

12. The kit according to any one of claims 10 to 11, wherein the chiauranib or the derivative thereof and the immune checkpoint inhibitor are placed in the same vessel or respectively placed in different vessels.

13. The kit according to any one of claims 10 to 12, wherein the chiauranib or the derivative thereof is in a dosage form for gastrointestinal administration, and is preferably an oral preparation; and the immune checkpoint inhibitor is in a dosage form for parenteral administration, and is preferably an injection preparation.

14. The kit according to any one of claims 10 to 13, further comprising instructions for use.

15. A method for treating a tumor, comprising the following steps: administering a therapeutically effective amount of the pharmaceutical composition according to claim 9 or the kit according to any one of claims 10 to 14 to a tumor patient in need.

16. The method according to claim 15, wherein the pharmaceutical composition according to claim 9 or the kit according to any one of claims 10 to 14 is administered to the tumor patient as a first-line treatment.

17. The method according to claim 15, wherein the pharmaceutical composition according to claim 9 or the kit according to any one of claims 10 to 14 is administered to the tumor patient as a second-line, third-line, fourth-line, fifth-line or sixth-line treatment.

18. The method according to claim 15, wherein the tumor patient has not been treated or has undergone at least one antitumor treatment.

19. The method according to claim 18, wherein the antitumor treatment comprises surgery, chemotherapy, radiotherapy, targeted therapy, and immunotherapy, or a combination thereof.

20. The method according to any one of claims 15 to 19, wherein the chiauranib or the derivative thereof and the immune checkpoint inhibitor are administered simultaneously, separately or sequentially.
